# EUROPÄISCHE PATENTSCHRIFT

(11) **EP 3 369 406 B1**
(45) Veröffentlichungstag und Bekanntmachung des Hinweises auf die Patenterteilung: **26.08.2026**
(21) Anmeldenummer: 18159507.5
(22) Anmeldetag: 01.03.2018
(51) Int. Cl.: A61F 9/007

(54) **GLAUKOM-DRAINAGE-IMPLANTAT**
GLAUCOMA DRAINAGE IMPLANT
IMPLANT DE DRAINAGE DE GLAUCOME

(30) Priorität: 03.03.2017 DE 102017104543
(43) Veröffentlichungstag der Anmeldung: 05.09.2018
(73) Patentinhaber: Nikolic, Stephan, 30167 Hannover (DE)
(72) Erfinder: Lubatschowski, Holger, 30989 Gehrden (DE); Nikolic, Stephan, 30167 Hannover (DE)
(74) Vertreter: Eisenführ Speiser

(56) Entgegenhaltungen:
- US-A- 5 171 213
- US-A1- 2015 257 931
- US-A1- 2016 058 615
- US-B2- 7 641 627

## Beschreibung

Die vorliegende Erfindung betrifft ein Glaukom-Drainage-Implantat mit einem Druckentlastungsventil über das Kammerwasser abgelassen werden kann.

Das Glaukom ist eine der häufigsten Erblindungsursachen weltweit. Es führt zu einem irreversiblen Untergang von retinalen Ganglienzellen. Der zu Grunde liegende intraokulare Druckanstieg muss, wenn Medikamente oder Laserbehandlungen versagen, durch einen operativen Eingriff angeglichen werden. Hier ist die Trabekulektomie die häufigste angewandte Methode. Ist diese jedoch fehlgeschlagen oder kontraindiziert, ist der Einsatz von Drainage-Implantaten Mittel der Wahl.

Generell lassen sich bei den derzeitigen Implantaten Systeme mit und ohne Ventil unterscheiden. Implantate ohne Ventil haben das zusätzliche Risiko einer postoperativen Hypotonie durch fehlenden Verschluss nach Drucksenkung. Das sogenannte Ahmed-Implantat ist ein aus dem Stand der Technik bekanntes Implantat mit Ventil, welches der Hypotonie vorbeugt. Der Langzeitverlauf zeigte aber im Vergleich mit der Trabekulektomie keine signifikanten Unterschiede.

Aus US2016/0058615A1 ist ein Glaukomimplantat bekannt, bei dem mittels eines Kanals das Kammerwasser einem Filterelement zugeführt wird und mittels eines Schlitzventils abgelassen wird. Das Schlitzventil dient der Regulierung des Flusses von dem Kammerinnenraum zu der äußeren Oberfläche des Auges. Es bleibt geschlossen, bis der intraokulare Druck einen Druck erreicht, bei dem das Ventil öffnet und den Abfluss von Kammerwasser freigibt oder verstärkt. Das Schlitzventil dieser vorbekannten Vorrichtung ist zur druckabhängigen Abflussregulierung ausgebildet, Es kann durch Eiweißablagerung verstopfen und ist nicht dazu ausgebildet, um in einem solchen Falle durch eine externe Einwirkung bewegt zu werden um diese Verstopfung aufzulösen. Aufgrund der Größenverhältnisse und der Gefahr einer Beschädigung der Implantatverankerung und Infektion ist eine mechanische Berührung des Ventils zum Zwecke einer solchen externen Betätigung nicht handhabbar und würde ein Risiko für den Patienten darstellen. Insbesondere ist im Sinne dieser Erfindung ein Ventil dieser Bauart mit einem Schlitz in einer mechanisch verformbaren Struktur nicht als ein Ventil zu verstehen, das einen Betätigungsbereich aufweist, mit dem eine Öffnung des Ventils bewirkt werden kann, da dies bei einem solchen Ventil weder vorgesehen noch praktikabel ist.

US 2015/257931 A1 beschreibt ein Ventil in Form einer Klappe, die gegen Kanalwände abschließt.

Es ist Aufgabe der vorliegenden Erfindung ein Glaukom-Drainage-Implantat zu schaffen, dass einen verbesserten Langzeitverlauf begünstigt.

Die Aufgabe wird gelöst durch ein Glaukom-Drainage-Implantat gemäß Anspruch 1 mit einem Druckentlastungsventil über das Kammerwasser abgelassen werden kann, wobei das Druckentlastungsventil eine Druckentlastungsklappe aufweist, die mit einer in einer Lederhaut einzubettenden Grundplatte des Implantats beweglich verbunden ist, wobei die Druckentlastungsklappe einen Betätigungsbereich aufweist, mittels dem die Druckentlastungsklappe im Zuge einer postoperativen Einwachsphase oder danach non-invasiv mobilisiert werden kann.

Die Erfindung schließt die Erkenntnis ein, dass als Reaktion auf den Implantat-Fremdkörper häufig eine fibrovaskuläre (durch Kollagenbildung hervorgerufene) Abkapselung des Implantates auftritt, die eine nicht unerhebliche Komplikation darstellt. Der menschliche Körper versucht nach Verletzungen, also auch nach operativen Eingriffen einen Verschluss des defekten Wundgebietes zu erreichen. Aus diesem Grunde werden im Bereich der operativen Verletzungen vermehrt Entzündungsmediatoren ausgesendet um eine Gewebeheilung, unter Umständen auch mit Defekten, zu bewirken. Durch die Bildung der Entzündungsmediatoren entstehen Fibrose (Bildung von Kollagenfasern) und vermehrte Protein- und Entzündungszellenbildung im Kammerwasser (im Auge gebildete Flüssigkeit zur Regulierung des Augeninnendruckes nebst Versorgung). Dies kann sowohl zu einem Verstopfen eines im Auge platzierten Glaukom-Drainage-Implantates führen als auch zum Zuwachsen einer operativ geschaffenen Skleraklappe, welche die Filtration gewährleisten soll. Dadurch wird der gewünschte Abfluss des Kammerwassers unterbunden, sodass ein weiterer Druckanstieg erfolgt.

Das erfindungsgemäße Glaukom-Drainage-Implantat bietet in Abkehr zum Stand der Technik den Vorteil, dass durch den in der Druckentlastungsklappe vorgesehenen Betätigungsbereich eine fibrotische Abkapselung unterbunden werden kann. Es ist nunmehr möglich, durch ein non-invasives Mobilisieren eine unerwünschte Funktionseinschränkung des Druckentlastungsventils zu vermindern oder gänzlich zu vermeiden. Vorteilhafterweise kann auf den Einsatz von Zellgiften wie Mitomycin C oder 5-Fluoruracil und damit Verhinderung der gefürchteten sterilen Endophthalmitis in der Spätphase verhindert werden.

Die Druckentlastungsklappe und die Grundplatte bilden erfindungsgemäß zusammen das Druckentlastungsventil. Die Druckentlastungsklappe wirkt dabei als Verschlussteil des Ventils. Über eine Relativbewegung der Druckentlastungsklappe zur Grundplatte wird das Ventil geöffnet oder geschlossen. Ein Öffnen der Druckentlastungsklappe im Sinne der Erfindung bedeutet, dass die Druckentlastungsklappe in einem bestimmten Bereich von der Grundplatte abgehoben wird, während sie in anderen Bereichen mit dieser beweglich verbunden bleibt.

In einer bevorzugten Ausgestaltung weist der der Betätigungsbereich einen thermomechanisch wirksamen Bereich auf, so dass die Druckentlastungsklappe non-invasiv mittels Temperaturänderung mobilisiert werden kann. Vorzugsweise ist der Betätigungsbereich in Form eines Formgedächtnismaterials bereitgestellt oder weist ein solches auf. Vorteilhafterweise weist das Formgedächtnismaterial einen Zweiwege-Memory-Effekt auf. Das Formgedächtnismaterial kann ein Formgedächtnispolymer oder eine Formgedächtnislegierung sein oder jeweils ein/eine solche aufweisen.

Unter einem Mobilisieren der Druckentlastungsklappe soll im Rahmen der vorliegenden Erfindung insbesondere ein Öffnen der Druckentlastungsklappe mittels Wärmeeinbringung in der Nähe der Druckentlastungsklappe und ein selbsttätiges Schließen der Druckentlastungsklappe bei Abkühlung verstanden werden. Ein selbsttätiges Schließen muss nicht notwendigerweise ein vollständiges Schließen der Druckentlastungsklappe bedeuten. Vielmehr kann vorgesehen sein, dass das Implantat bzw. die Druckentlastungsklappe bei Abkühlen je nach Anforderung einen leicht geöffneten Zustand annimmt, um Kammerwasser hinreichend abfließen zu lassen.

Unter einem Mobilisieren der Druckentlastungsklappe soll im Rahmen der vorliegenden Erfindung - bezogen auf einen als thermomechanisch wirksamen Bereich ausgebildeten Betätigungsbereich - insbesondere ein Öffnen der Druckentlastungsklappe mittels einer wirksam in den Bereich eingebrachten Wärmemenge, und ein selbsttätiges Schließen der Druckentlastungsklappe nach Entzug der Wärmemenge aus dem thermomechanisch wirksamen Bereich verstanden werden. Alternativ kann ein Öffnen der Druckentlastungsklappe mittels einer wirksam dem Bereich entzogenen Wärmemenge, und ein selbsttätiges Schließen der Druckentlastungsklappe durch entsprechende Wiederzufuhr der Wärmemenge in den Bereich realisiert werden.

Ein selbsttätiges Schließen muss nicht notwendigerweise ein vollständiges Schließen der Druckentlastungsklappe bedeuten. Vielmehr kann vorgesehen sein, dass das Implantat bzw. die Druckentlastungsklappe bei menschlicher Körpertemperatur je nach Anforderung einen leicht geöffneten Zustand annimmt, um Kammerwasser hinreichend abfließen zu lassen.

In einer bevorzugten Ausgestaltung kann die Druckentlastungsklappe des Glaukom-Drainage-Implantats einen Betätigungsbereich aufweisen, der als thermomechanisch wirksamer Bereich ausgebildet ist. Dafür kann vorteilhafterweise eine Formgedächtnislegierung verwendet werden. Dies hat insbesondere Sicherheitsvorteile. So kann die Druckentlastungsklappe durch eine eingebrachte Wärmemenge aktiviert und geöffnet werden.

In einer bevorzugten Ausgestaltung ist das Implantat frei von einem Drainageschlauch.

Es hat sich als vorteilhaft herausgestellt, wenn der thermomechanische Bereich als FormGedächtnis-Legierung ausgebildet ist. Der thermomechanische Bereich, insbesondere ein als Metallplättchen ausgebildeter thermomechanischer Bereich, kann vollständig in der Druckentlastungsklappe eingeschlossen sein. Der thermoelastische Bereich kann beispielsweise NiTiNol aufweisen oder eine Metalllegierung aufweisen. Vorteilhafterweise kann der als thermomechanisch wirksame Bereich in Form eines Formgedächtnismaterials vollständig in der Druckentlastungsklappe eingeschlossen sein.

Es hat sich als vorteilhaft herausgestellt, wenn das Implantat scheibenförmig ausgebildet ist. Das Implantat weist vorzugsweise eine Dicke von höchstens 0,8 mm, insbesondere 0,6 mm und/oder eine Breite bzw. einen Durchmesser von höchstens 6 mm auf. Derart kann das Implantat nah des Limbusbereichs bzw. des Schlemm-Kanals platziert werden und auf einer aufwendigen Drainageleitung verzichtet werden.

In einer weiteren bevorzugten Ausgestaltung ist die Druckentlastungsklappe über ein Festkörpergelenk beweglich mit der Grundplatte verbunden. Dies ermöglicht eine besonders kompakte und wartungsarme Ausgestaltung des Implantats. Druckentlastungsklappe und Grundplatte können einstückig miteinander ausgebildet sein.

Es hat sich als besonders vorteilhaft herausgestellt, wenn die Grundplatte stufenförmig ausgebildet ist. Durch eine stufenförmige Ausbildung der Grundplatte, wobei die Abstufung der Grundplatte auf eine radiale Richtung des Auges bezogen ist, begünstigt die Vermeidung einer Verklebung von Bindehaut oder Tenon-Kapsel und Druckentlastungsklappe.

In einer weiteren bevorzugten Ausgestaltung ist die Druckentlastungsklappe durch die Grundplatte umrandet. Vorzugsweise ist die Grundplatte kreisringförmig ausgebildet, wobei die Druckentlastungsklappe kreisförmig ausgebildet ist. Alternativ kann die Grundplatte rahmenförmig ausgebildet sein. In diesem Fall weist die Druckentlastungsklappe eine rechteckförmige oder quadratische Außenkontur auf.

Es hat sich als vorteilhaft herausgestellt, wenn die Druckentlastungsklappe Silikon aufweist, besonders bevorzugt besteht die Druckentlastungsklappe - abgesehen von dem thermomechanisch wirksamen Bereich - aus Silikon. Gleichsam kann das gesamte Glaukom-Drainage-Implantat - abgesehen von dem thermomechanisch wirksamen Bereich - aus Silikon bestehen.

Es hat sich als vorteilhaft herausgestellt, wenn die Druckentlastungsklappe auf ihrer der Hornhaut zugewandten Seite eine Oberflächenstrukturierung aufweist. Grundsätzlich kann die Oberflächenstrukturierung aber auch auf der der Hornhaut abgewandten Seite vorgesehen sein, oder auf beiden Seiten.

In einer besonders bevorzugten Ausgestaltung ist das Druckentlastungsventil ausgebildet, Kammerwasser erst oberhalb eines Augeninnendrucks von 15 mmHg (etwa 20mBar, also 2 kPa) abzulassen. Mit anderen Worten ist das Druckentlastungsventil ausgebildet, den Augeninnendruck zu regeln.

Die Aufgabe wird ebenfalls gelöst durch ein Verfahren zum Implantieren eines Glaukom-Drainage-Implantats in ein menschliches oder tierisches Auge mit den Schritten:
- Anlegen eines Lederhautbetts in einer Lederhaut des Auges, wobei das Lederhautbett ein Drainagekanal aufweist;
- Einsetzen eines vorbeschriebenen Glaukom-Drainage-Implantats in das Lederhautbett derart, dass die Grundplatte das Lederhautbett wasserdicht abschließt und
- Mobilisieren der Druckentlastungsklappe mittels einer einen Temperaturgradienten erzeugenden Energiequelle im Zuge oder nach einer postoperativen Einwachsphase des Implantates.

Das Verfahren kann durch die mit Bezug auf das Glaukom-Drainage-Implantat beschriebenen Merkmale in entsprechender Weise vorteilhaft ausgestaltet sein.

So kann die Druckentlastungsklappe des im Verfahren verwendeten Glaukom-Drainage-Implantats einen Betätigungsbereich aufweisen, der als thermomechanisch wirksamer Bereich ausgebildet ist.

In einer bevorzugten Ausgestaltung des Verfahrens erfolgt eine Wärmemengenzufuhr (Temperaturerhöhung des thermomechanisch wirksamen Bereichs) durch Einstrahlung von Licht, vorzugsweise infrarotem Licht, welches vom umliegenden Gewebe nicht oder wenig absorbiert wird. Die Absorption der Strahlung durch das Material des Implantats oder Teile des Implantats führt zu der Temperaturerhöhung. Die thermische Wirkung kann lokal in einer gewünschten Weise konzentriert werden, indem eine für die verwendete Wellenlänge stark absorbierende Farbgebung in den Bereichen des Implantats vorgesehen ist, die sich erwärmen sollen.

Alternativ oder zusätzlich kann eine Temperaturerhöhung auch induktiv über einen elektrischen Strom erfolgen. In diesem Fall ist das Formgedächtnismaterial elektrisch leitfähig ausgestaltet, sodass über ein von aßen aufgebrachtes hochfrequentes Magnetfeld ein Wirbelstrom darin induziert werden kann, der die Erwärmung bewirkt. Dabei kann, um eine Konzentration der Feldlinien zu bewirken, ein Polschuh eingesetzt und entsprechend angeordnet werden.

Weitere Vorteile ergeben sich aus der folgenden Figurenbeschreibung. In den Figuren sind verschiedene Ausführungsbeispiele der vorliegenden Erfindung dargestellt. Die Figuren, die Beschreibung und die Ansprüche enthalten zahlreiche Merkmale in Kombination. Der Fachmann wird die Merkmale zweckmässigerweise auch einzeln betrachten und zu sinnvollen weiteren Kombinationen zusammenfassen.

Es zeigen:
- Fig. 1a: eine geschnittene Seitenansicht eines ersten bevorzugten Ausführungsbeispiels des erfindungsgemäßen Glaukom-Drainage-Implantats in Implantationslage in einem Auge;
- Fig. 1b: eine schematische Draufsicht eines bevorzugten Ausführungsbeispiels des erfindungsgemäßen Glaukom-Drainage-Implantats,
- Fig. 2a-c: eine geschnittene Seitenansicht eines Glaukom-Drainage-Implantats des Standes der Technik in einer üblichen Position nach Implantation mit nachteiligem Verlauf; und
- Fig. 3a,b: ein zweites bevorzugtes Ausführungsbeispiel des erfindungsgemäßen Glaukom-Drainage-Implantats nach Implantation mit vorteilhaftem Verlauf.

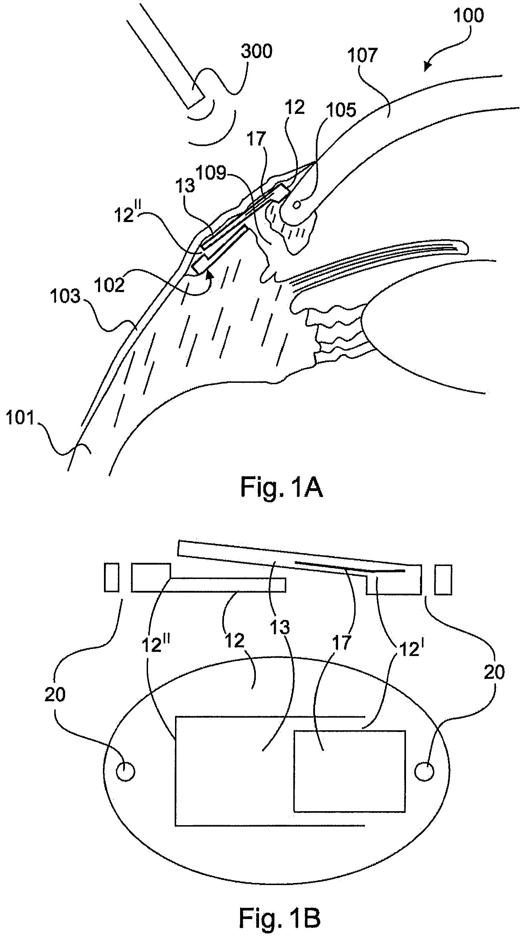

Eine bevorzugte Ausgestaltung des erfindungsgemäßen Glaukom-Drainage-Implantats wird nun beispielhaft mit Bezug auf Fig. 1a und b erläutert.

Ein menschliches Auge 100 ist in Fig. 1a in einem Schnitt nahe des Limbusbereichs gezeigt. Im oberen Teil der Fig. 1 ist die Hornhaut 107 erkennbar, in deren unteren Teil der Schlemm-Kanal 105 eingezeichnet ist.

In der Lederhaut 101 ist ein Lederhautbett 102 mit einem Drainagekanal 109 ausgearbeitet. Das Lederhautbett 102 kann beispielsweise mittels eines Lasers erzeugt werden oder aber auch händisch präpariert werden.

Eingebettet in das Lederhautbett 102 ist ein Glaukom-Drainage-Implantat 10. Das Implantat 10 wirkt als Druckentlastungsventil, über das Kammerwasser abgelassen werden kann. Das als Druckentlastungsventil wirkende Implantat 10 weist eine Druckentlastungsklappe 13 auf, die mit einer in der Lederhaut 101 eingebetteten Grundplatte 12 des Implantats 10 beweglich verbunden ist.

Wie der Fig. 1a entnommen werden kann, ist die Grundplatte 12 derart in das Lederhautbett 102 eingepasst, dass dieses seitlich zu der Lederhaut 101 abgedichtet ist, so dass, abgesehen vom Weg über den Drainagekanal 109 und die Druckentlastungsklappe 13, kein Kammerwasser abfließen kann.

Der Drainagekanal 109 ist am Boden des Lederhautbetts 102 ausgearbeitet. Das Implantat 10 in Fig. 1a dargestellten Ausführungsbeispiel ist scheibenförmig ausgebildet und hier in Schnittdarstellung gezeigt. Das Implantat weist eine Dicke von höchstens 0,8 mm (in radialer Richtung bezogen auf das Auge 100) auf und eine Breite von höchstens 6 mm (bezogen auf die Tangentialrichtung).

Die kreisringförmig ausgebildete Grundplatte 12 umschließt seitlich die Druckentlastungsklappe 13. Die Druckentlastungsklappe 13 ist über einen Anschlagsbereich 12' beweglich mit der Grundplatte 12 verbunden. Der Anschlagsbereich dient als Festkörpergelenk. Auf der gegenüberliegenden Seite findet sich der Öffnungsbereich 12" der Druckentlastungsklappe 13. Die Druckentlastungsklappe 13 und die Grundplatte 12 bilden das Druckentlastungsventil. Beim Öffnen der Druckentlastungsklappe 13 wird diese im Öffnungsbereich 12" von der Grundplatte abgehoben, während sie im Anschlagsbereich 12' mit der Grundplatte beweglich verbunden bleibt.

Wie ebenfalls der Figur 1 entnommen werden kann, ist die Grundplatte 12, bezogen auf die Radialrichtung R, stufenförmig ausgebildet, wobei die Grundplatte 12 im Anschlagsbereich 12' eine größere Dicke aufweist, als in dem Anschlagsbereich 12' gegenüberliegenden Öffnungsbereich 12".

Im in Fig. 1a gezeigten Zustand ist die Druckentlastungsklappe 13 leicht geöffnet, so dass eine Kammerwassermenge durch den Drainagekanal 109 und das als Ventil wirkende Implantat abfließen kann.

Wie ebenfalls aus Fig. 1a ersichtlich ist, ragt die Druckentlastungsklappe 13 zumindest abschnittsweise über den Drainagekanal 109 hinweg, so dass mittels der Druckentlastungskappe 13 der Drainagekanal verschlossen bzw. im Überdruckfall freigegeben werden kann. Das Druckentlastungsventil wirkende Implantat ist ausgebildet, dass Kammerwasser erst oberhalb eines Augeninnendrucks von 20 mBar, also 2 kPa, abzulassen.

Wie aus Fig. 1b ersichtlich, weist die Druckentlastungsklappe 13 einen thermomechanisch wirksamen Bereich 17 aus, so dass die Druckentlastungsklappe 13 im Zuge einer postoperativen Einwachsphase non-invasiv mittel Wärme (erzeugt durch Lasereinstrahlung oder induktiv) mobilisiert werden kann.

Der thermomechanisch wirksame Bereich 17 ist in dem vorliegend dargestellten Ausführungsbeispiel als Metallplättchen aus einer Formgedächtnis-Legierung ausgebildet und das vollständig in der Druckentlastungsklappe 13 und Anschlagsbereich 12' eingeschlossen ist. Die Druckentlastungsklappe 13 und der Anschlagsbereich 12' bestehen - abgesehen von den thermomechanischen Bereichen - aus Silikon.

Über die angebrachten Ösen 20 kann das Implantat mit einem Faden an der Sklera fixiert werden.

Wird beispielsweise infrarote Laserstrahlung in Richtung des thermomechanisch wirksamen Bereichs 17 eingestrahlt, so wirkt ein Drehmoment auf die Druckentlastungsklappe 13 ein, so dass diese sich in radialer Richtung, in Fig. 1a nach oben, bewegt und derart mobilisiert und geöffnet wird.

Kühlt der thermomechanisch wirksame Bereich des Ventils auf Körpertemperatur ab, so schließt sich die Druckentlastungsklappe 13 selbsttätig aufgrund der entsprechenden Rückstellung des als Festkörpergelenk dienenden Anschlagsbereich 12' über das die Druckentlastungsklappe 13 mit der Grundplatte 12 verbunden ist. Sofern der Augeninnendruck kleiner als 20 mBar, also 2 kPa, ist, verschließt sich die Druckentlastungsklappe 13 vollständig.

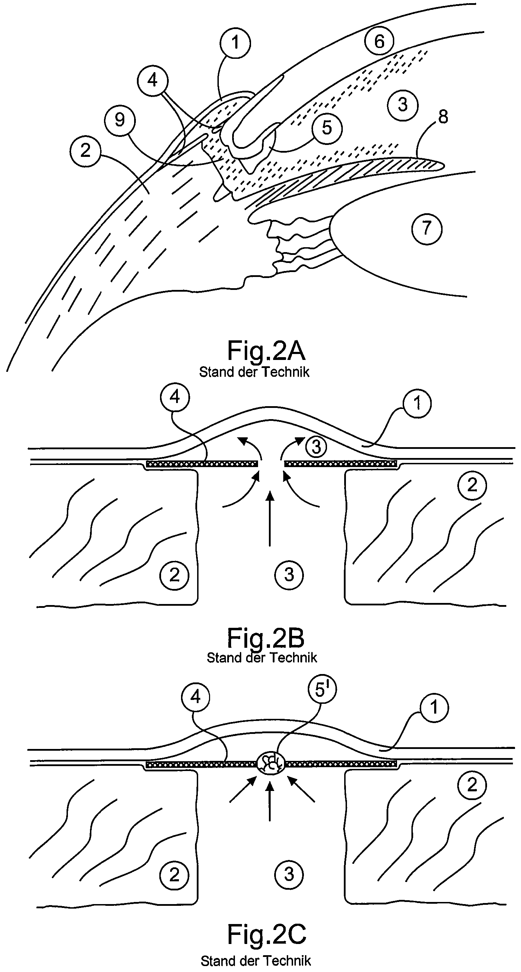

Fig. 2A - C zeigen ein Glaukom-Drainage-Implantat des Standes der Technik in einer üblichen Position nach Implantation mit nachteiligem Verlauf.

Dabei zeigt Fig. 2A die übliche Position eines Implantats 4 im Auge zur Dränierung der Kammerwassers 3. Dabei wurden die Sklera 2 und das Trabekelwerk 5 chirurgisch perforiert 9. Das aus der Augenvorderkammer durch eine Implantat-Öffnung fließende Kammerwasser 3 wird von der Bindehaut 1 aufgehalten, unter der sich üblicherweise eine entsprechende Blase bildet. Weiter dargestellt sind Hornhaut 6 des Auges, sowie die Iris 8 und die Augenlinse 7.

In Fig. 2B ist der Zustand unmittelbar nach Implantation des Implantats dargestellt. Das Implantat 4 befindet sich zwischen Bindehaut 1 und Sklera 2. Die Sklera ist durch eine chirurgisch herbeigeführte Öffnung unterbrochen, in der das Kammerwasser 3 durch eine Öffnung des Implantats nach außen unter die Bindehaut gelangt und dort eine Blase bildet.

Fig. 2C stellt den Zustand einige Wochen nach erfolgter Implantation dar. Die Öffnung des Implantats ist durch Fibrose-Ablagerungen 5' verstopft. Ein freies Abfließen von Kammerwasser 3 findet nicht mehr statt.

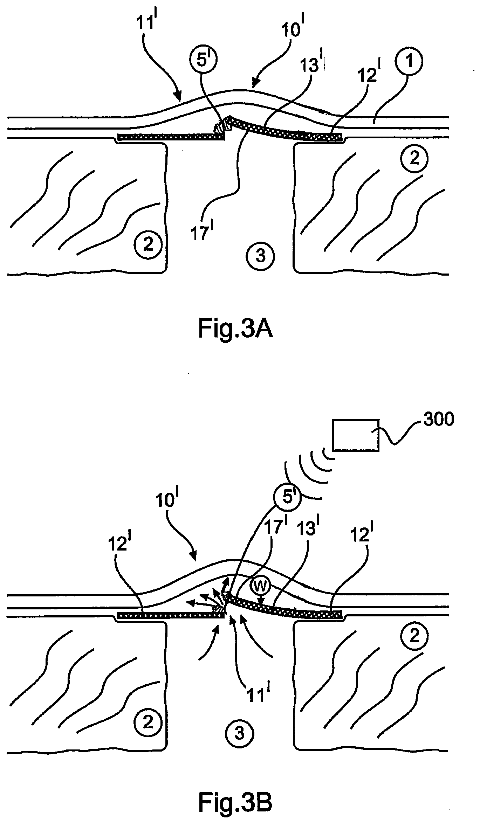

Fig. 3A - B zeigen nunmehr ein zweites bevorzugtes Ausführungsbeispiel des erfindungsgemäßen Glaukom-Drainage-Implantats 10' nach Implantation mit vorteilhaftem Verlauf.

Wie der Fig. 3A entnommen werden kann, wirkt das Glaukom-Drainage-Implantat 10' als Druckentlastungsventil 11' über das Kammerwasser 3 abgelassen werden kann. Das Druckentlastungsventil 11' weist eine Druckentlastungsklappe 13' auf, die mit einer in einer Lederhaut 2 einzubettenden Grundplatte 12' des Implantats 10' beweglich verbunden ist. Die Druckentlastungsklappe 13' weist einen Betätigungsbereich in Form eines thermomechanisch wirksamen Bereichs mit einem Formgedächtnismaterial 17' mit Zweiwege-Memory-Effekt auf, mittels dem die Druckentlastungsklappe 13' im Zuge einer postoperativen Einwachsphase oder danach non-invasiv mobilisiert werden kann. Die Druckentlastungsklappe 13 ist über den Anschlagsbereich der als Festkörpergelenk dient beweglich mit der Grundplatte 12' verbunden. Die Druckentlastungsklappe 13 und die Grundplatte 12 bilden das Druckentlastungsventil. Beim Öffnen der Druckentlastungsklappe 13' wird diese in einem Öffnungsbereich von der Grundplatte abgehoben, während sie im Anschlagsbereich mit der Grundplatte beweglich verbunden bleibt.

In Fig. 3A ist eine durch die Druckentlastungsklappe 13' freizugebende Öffnung des Implantats 10' durch Fibrose-Ablagerungen 5' verstopft.

Fig. 3B zeigt nunmehr den Zustand, nachdem die bewegliche Druckentlastungsklappe 13' ein- oder mehrfach bewegt wurde. Die Bewegung hat dafür gesorgt, dass die Fibrose-Ablagerungen aufgerissen sind und den Weg des Kammerwassers 3 nach außen (Pfeilrichtung) freigelegt wurde.

Ein Mobilisieren der Druckentlastungsklappe 13' erfolgt dabei mittels einer Infrarotquelle 300 über die eine Wärmemenge W in den thermomechanisch wirksamen Bereich 17' mit dem Formgedächtnismaterial eingetragen wird.

### Bezugszeichenliste

- 3: Kammerwasser
- 5: Trabekelwerk
- 5': Fibrose-Ablagerungen
- 6, 107: Hornhaut
- 7: Augenlinse
- 8: Iris
- 10, 10': Glaukom-Drainage-Implantat
- 11, 11': Teil des Implantats, der als Druckentlastungsventil wirkt
- 12: Grundplatte
- 12': Anschlagsbereich
- 12": Öffnungsbereich
- 13, 13': Druckentlastungsklappe
- 17, 17': thermomechanisch wirksamer Bereich (Formgedächtnismaterial)
- 20: Öse
- 100: Auge
- 2, 101: Lederhaut (Sklera)
- 102: Lederhautbett
- 1, 103: Bindehaut
- 105: Schlemm-Kanal
- 9, 109: chirurgische Perforation, Drainagekanal
- 300: Infrarotquelle
- W: Wärmemenge

## Patentansprüche

1. Glaukom-Drainage-Implantat (10, 10') mit einem Druckentlastungsventil (11) über das Kammerwasser abgelassen werden kann,
wobei das Druckentlastungsventil (11) eine Druckentlastungsklappe (13) aufweist, die mit einer in einer Lederhaut (101) einbettbaren Grundplatte (12) des Implantats (10, 10') beweglich verbunden ist, wobei die Druckentlastungsklappe (13) einen Betätigungsbereich aufweist, mittels dem die Druckentlastungsklappe (13) im Zuge einer postoperativen Einwachsphase oder danach non-invasiv mobilisiert werden kann,
**dadurch gekennzeichnet, dass** die Druckentlastungsklappe (13) und die Grundplatte (12) das Druckentlastungsventil bilden.

2. Implantat (10, 10') nach Anspruch 1,
**dadurch gekennzeichnet, dass** der Betätigungsbereich einen thermomechanisch wirksamen Bereich (17) aufweist, so dass die Druckentlastungsklappe (13) non-invasiv mittels Temperaturänderung mobilisiert werden kann.

3. Implantat (10, 10') nach Anspruch 2,
**dadurch gekennzeichnet, dass** der Betätigungsbereich einen thermomechanisch wirksamen Bereich (17) in Form eines Formgedächtnismaterials (17') mit Zweiwege-Memory-Effekt aufweist.

4. Implantat (10, 10') nach einem der vorangehenden Ansprüche,
**dadurch gekennzeichnet, dass** das Implantat (10, 10') scheibenförmig mit einer Dicke von höchstens 0,8 mm ausgebildet ist.

5. Implantat (10, 10') nach einem der vorangehenden Ansprüche,
**dadurch gekennzeichnet, dass** das Implantat (10, 10') scheibenförmig mit einer Dicke von höchstens 0,6 mm ausgebildet ist.

6. Implantat (10, 10') nach einem der vorangehenden Ansprüche,
**dadurch gekennzeichnet, dass** die Druckentlastungsklappe (13) über einen Anschlagsbereich (12'), der als Festkörpergelenk dient, beweglich mit der Grundplatte (12) verbunden ist.

7. Implantat (10, 10') nach einem der vorangehenden Ansprüche,
**dadurch gekennzeichnet, dass** die Grundplatte (12) stufenförmig ausgebildet ist, wobei die Grundplatte (12) in einem Anschlagsbereich (12') der Druckentlastungsklappe (13) eine größere Dicke aufweist als in einem dem Anschlagsbereich (12') gegenüberliegenden Öffnungsbereich (12") der Druckentlastungsklappe (13).

8. Implantat (10, 10') nach einem der vorangehenden Ansprüche,
**dadurch gekennzeichnet, dass** die Druckentlastungsklappe (13) durch die Grundplatte (12) umrandet ist.

9. Implantat (10, 10') nach einem der vorangehenden Ansprüche,
**dadurch gekennzeichnet, dass** die Druckentlastungsklappe (13) Silikon aufweist.

10. Implantat (10, 10') nach einem der vorangehenden Ansprüche,
**dadurch gekennzeichnet, dass** die Druckentlastungsklappe (13) auf ihrer der Hornhaut (107) zugewandten Seite eine Oberflächenstrukturierung aufweist.

11. Implantat (10, 10') nach einem der vorangehenden Ansprüche,
**dadurch gekennzeichnet, dass** das Druckentlastungsventil (11) zur Regelung des Augeninnendrucks derart ausgebildet ist, dass das Druckentlasungsventil erst oberhalb eines Augeninnendrucks von 20 mBar (15 mmHg) öffnet, indem die Druckentlastungsklappe durch den Augeninnendruck von der Grundplatte abgehoben wird, um Kammerwasser abzulassen.

## Claims

1. A glaucoma drainage implant (10, 10') having a pressure relief valve (11) through which aqueous humor can be drained,
wherein the pressure relief valve (11) comprises a pressure relief flap (13) that is movably connected to a base plate (12) of the implant (10, 10') that can be embedded in a sclera (101), wherein the pressure relief flap (13) has an actuation region by means of which the pressure relief flap (13) can be mobilized non-invasively during a postoperative ingrowth phase or thereafter,
**characterized in that** the pressure relief flap (13) and the base plate (12) form the pressure relief valve.

2. Implant (10, 10') according to claim 1,
**characterized in that** the actuation region comprises a thermomechanically effective region (17), such that the pressure relief flap (13) can be mobilized non-invasively by means of a temperature change.

3. Implant (10, 10') according to claim 2,
**characterized in that** the actuating region comprises a thermomechanically active region (17) in the form of a shape-memory material (17') with a two-way memory effect.

4. Implant (10, 10') according to one of the preceding claims,
**characterized in that** the implant (10, 10') is disc-shaped with a thickness of at most 0.8 mm.

5. Implant (10, 10') according to one of the preceding claims,
**characterized in that** the implant (10, 10') is disc-shaped with a thickness of at most 0.6 mm.

6. An implant (10, 10') according to any one of the preceding claims,
**characterized in that** the pressure relief flap (13) is movably connected to the base plate (12) via a stop region (12') that serves as a solid-state hinge.

7. Implant (10, 10') according to one of the preceding claims,
**characterized in that** the base plate (12) is stepped, wherein the base plate (12) has a greater thickness in a stop region (12') of the pressure relief flap (13) than in an opening region (12") of the pressure relief flap (13).

8. Implant (10, 10') according to any one of the preceding claims,
**characterized in that** the pressure relief flap (13) is surrounded by the base plate (12).

9. Implant (10, 10') according to one of the preceding claims,
**characterized in that** the pressure relief flap (13) comprises silicone.

10. Implant (10, 10') according to one of the preceding claims,
**characterized in that** the pressure relief flap (13) has a surface texture on its side facing the cornea (107).

11. Implant (10, 10') according to one of the preceding claims,
**characterized in that** the pressure relief valve (11) for regulating intraocular pressure is designed such that the pressure relief valve opens only above an intraocular pressure of 20 mBar (15 mmHg), whereby the pressure relief flap is lifted off the base plate by the intraocular pressure to allow aqueous humor to drain.

## Revendications

1. Implant de drainage de glaucome (10, 10') avec une valve de décharge de pression (11) par le biais de laquelle de l'humeur aqueuse peut être évacuée,
dans lequel la valve de décharge de pression (11) présente un clapet de décharge de pression (13), qui est relié de manière mobile à une plaque de base (12) de l'implant (10, 10') pouvant être incorporée dans une sclérotique (101), dans lequel le clapet de décharge de pression (13) présente une zone d'actionnement, au moyen de laquelle le clapet de décharge de pression (13) peut être mobilisé de manière non invasive au cours d'une phase de croissance interne post opératoire ou par après,
**caractérisé en ce que** le clapet de décharge de pression (13) et la plaque de base (12) forment la valve de décharge de pression.

2. Implant (10, 10') selon la revendication 1,
**caractérisé en ce que** la zone d'actionnement présente une zone efficace du point de vue thermomécanique (17) de sorte que le clapet de décharge de pression (13) peut être mobilisé de manière non invasive par changement de température.

3. Implant (10, 10') selon la revendication 2,
**caractérisé en ce que** la zone d'actionnement présente une zone efficace du point de vue thermomécanique (17) sous la forme d'un matériau à mémoire de forme (17') avec effet mémoire bidirectionnel.

4. Implant (10, 10') selon l'une quelconque des revendications précédentes,
**caractérisé en ce que** l'implant (10, 10') est réalisé en forme de disque avec une épaisseur de maximum 0,8 mm.

5. Implant (10, 10') selon l'une quelconque des revendications précédentes,
**caractérisé en ce que** l'implant (10, 10') est réalisé en forme de disque avec une épaisseur de maximum 0,6 mm.

6. Implant (10, 10') selon l'une quelconque des revendications précédentes,
**caractérisé en ce que** le clapet de décharge de pression (13) est relié de manière mobile à la plaque de base (12) par le biais d'une zone de butée (12'), qui sert d'articulation à corps rigide.

7. Implant (10, 10') selon l'une quelconque des revendications précédentes,
**caractérisé en ce que** la plaque de base (12) est réalisée de manière étagée, dans lequel la plaque de base (12) présente dans une zone de butée (12') du clapet de décharge de pression (13) une épaisseur supérieure à celle dans une zone d'ouverture (12") du clapet de décharge de pression (13) opposée à la zone de butée (12').

8. Implant (10, 10') selon l'une quelconque des revendications précédentes,
**caractérisé en ce que** le clapet de décharge de pression (13) est bordé par la plaque de base (12).

9. Implant (10, 10') selon l'une quelconque des revendications précédentes,
**caractérisé en ce que** le clapet de décharge de pression (13) présente du silicone.

10. Implant (10, 10') selon l'une quelconque des revendications précédentes,
**caractérisé en ce que** le clapet de décharge de pression (13) présente sur son côté tourné vers la cornée (107) une structuration superficielle.

11. Implant (10, 10') selon l'une quelconque des revendications précédentes,
**caractérisé en ce que** la valve de décharge de pression (11) est réalisée pour la régulation de la pression intraoculaire de telle sorte que la valve de décharge de pression ne s'ouvre que lorsqu'une pression intraoculaire est supérieure à 20 mBar (15 mmHg), par le fait que le clapet de décharge de pression est soulevé de la plaque de base par la pression intraoculaire, pour évacuer de l'humeur aqueuse.
